Europäisches Patentamt
European Patent Office
Office européen des brevets

(19)

(11) Publication number: 0 095 888
A2

(12) EUROPEAN PATENT APPLICATION

(21) Application number: 83303002.6

(22) Date of filing: 24.05.83

(51) Int. Cl.³: C 07 D 213/89, A 01 N 43/40

(30) Priority: 28.05.82 JP 91929/82
24.11.82 JP 206310/82

(43) Date of publication of application: 07.12.83
Bulletin 83/49

(84) Designated Contracting States: CH DE FR GB IT LI NL

(71) Applicant: SUMITOMO CHEMICAL COMPANY, LIMITED,
15 Kitahama 5-chome Higashi-ku, Osaka-shi Osaka-fu (JP)

(72) Inventor: Nakayama, Koji, No. 3-64, Kirehigashi 2-chome Hirano-ku, Osaka-shi Osaka (JP)
Inventor: Yoshida, Ryo, No. 5-19, Higashiuneno-aza-jizoyama, Kawanishi-shi Hyogo (JP)
Inventor: Matsumoto, Hiroshi, No. 10-2-211, Sonehigashimachi 2-chome, Toyonaka-shi Osaka (JP)

(74) Representative: Moore, Anthony John et al, Gee & Co.
Chancery House Chancery Lane, London WC2A 1QU (GB)

(54) **Benzylsulfinyl pyridine-N-oxides.**

(57) A benzylsulfinyl pyridine-N-oxide of the formula:

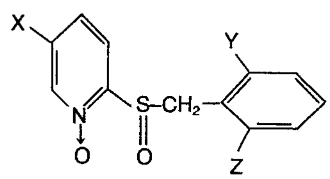

(I)

wherein X is a chlorine atom or a bromine atom, and Y and Z are each independently a chlorine atom or a fluorine atom, is usefui as the active ingredient of a herbicide and may be prepared by oxidizing the corresponding benzylthiopyridine-N-oxide.

EP 0 095 888 A2

Benzylsulfinyl pyridine-N-oxides

This invention relates to benzylsulfinyl pyridine-N-oxides represented by the following general formula (I):

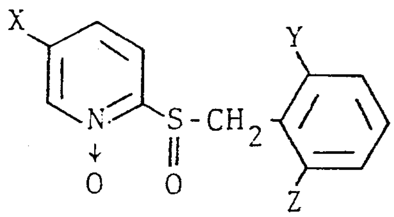

(I)

wherein X is a chlorine atom or a bromine atom; and Y and Z, which may be the same or different, are each a chlorine atom or a fluorine atom; a process for producing such compounds; and herbicides containing one or more of such compounds as active ingredients.

It is disclosed in U.S. Patent 4,019,893 and European Patent Publication No. 0036638A2 that some pyridine-N-oxides, e.g., 2-(2,6-dichlorobenzylsulfinyl)-pyridine-N-oxide and 5-bromo-2-(2,6-dichlorobenzylsulfonyl)-pyridine-N-oxide, are useful as active ingredients of herbicides. However, these known compounds are not always satisfactory as herbicides.

The compound of this invention have herbicidal activity against various weeds which present a problem in the pre-emergence soil treatment of upland fields, such as broad-leafed weeds (e.g., wild buckwheat (*Polygonum convolvulus*), chickweed (*Stellaria media*), radish (*Raphanus sativus*), wild mustard (*Sinapis arvensis*), hemp sesbania (*Sesbania exaltata*), sicklepod (*Cassia obtusifolia*), velvetleaf (*Abutilon theophrasti*), prickly sida (*Sida spinosa*), bedstraw (*Galium aparine*),

ivyleaf morning glory (Ipomoea hederacea), tall morning glory (Ipomoea purpurea), cocklebur (Xanthium strumarium) and corn marigold (Chrysanthemum segetum); gramineous weeds (e.g., Japanese millet (Echinochloa frumentacea), barnyard grass (Echinochloa crus-galli), green foxtail (Setaria viridis), large crabgrass (Digitaria sanguinalis), annual bluegrass (Poa annua), black grass (Alopecurus myosuroides), oat (Avena sativa), wild oat (Avena fatua), Johnsongrass (Sorghum halepense), quackgrass (Agropyron repens), bermudagrass (Cynodon dactylon) and fall panicum (Panicum dichotomiflorum); Commelina weeds (e.g., dayflower (Commelina communis); and sedge weeds (e.g., Cyperus esculentus. Moreover, some of the compounds of this invention are not substantially phytotoxic against main crops such as soybeans (Glycine max), cotton (Gossypium sp.), barley (Hordeum vulgare) and rice (Oryza sativa).

Furthermore, the compounds of this invention, without being substantially phytotoxic against rice (Oryza sativa), have herbicidal activity against various weeds which give rise to problems in paddy fields, such as gramineous weeds, e.g., barnyardgrass (Echinochloa orysicola); broadleafed weeds, e.g., false pimpernel (Lindernia procumbens), and spike-flowered rotala (Rotala indica); sedge weeds, e.g., bulrush (Scirpus juncoides) and slender spikerush (Eleocharis acicularis); and weeds found in paddy fields, e.g., Japanese ribbon wapato (Sagittaria pygmaea).

The compounds of this invention can be prepared by oxidizing benzylthiopyridine-N-oxide represented by the following general formula (II):

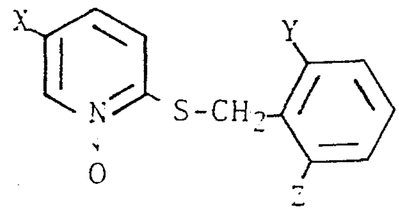

(II)

wherein X, Y and Z are as defined above, with an oxidizing agent.

Suitable examples of the oxidizing agents which may be used include inorganic peroxides such as hydrogen peroxide and sodium metaperiodate; aliphatic peroxides such as peracetic acid; and aromatic peroxides such as metachloroperbenzoic acid.

The use of solvent is not essential, but when used, the solvent should be chosen depending upon the oxidizing agent employed. For example, it is suitable to use water, glacial acetic acid or acetone with hydrogen peroxide; and a halogenated hydrocarbon (e.g., chloroform or methylene chloride or an ether (e.g., diethyl ether or dioxane) with an aromatic peroxide. Further, when an aliphatic peroxide is used, it is desirable to use the oxidizing agent *per se* in excess.

The oxidizing agent is usually used in an amount of from 0.5 to 1.5 equivalents, preferably from 0.95 to 1.2 equivalents, with respect to the benzylthiopyridine-N-oxide. The reaction temperature which may be employed is from the freezing point to the boiling point of the solvent used, and preferably from 0°C to the boiling point of the solvent. The reaction time which may be employed is from 30 minutes to 10 hours. After

completion of the reaction, the reaction mixture is treated in a conventional manner. If desired, the product may be purified by recrystallization, column chromatography, or the like.

The benzylthiopyridine-N-oxide of the general formula (II) may be obtained according to, for example, the method disclosed in European Patent Publication No. 0036638A2.

The preparation of compounds of this invention will now be illustrated in the following Examples.

EXAMPLE 1

Preparation Compound (1):

3.2 g of 5-chloro-2-(2,6-dichlorobenzylthio)pyridine-N-oxide were dissolved in 50 ml of chloroform, and a solution of 2.5 g of metachloroperbenzoic acid (content: 70%) dissolved in 30 ml of chloroform was added dropwise thereto at 5 to 10°C over 10 minutes. The mixture was stirred at 5 to 10°C for an additional 2 hours. The resulting mixture was washed twice with potassium carbonate solution and dried over magnesium sulfate. The chloroform was evaporated and the residue was recrystallized from 1 : 1 benzene-hexane to obtain 3.2 g of 5-chloro-2-(2,6-dichlorobenzylsulfinyl)pyridine-N-oxide (Compound (1)). Yield, 95%. M.P., 176.5-177.5°C. H-NMR in $CDCl_3$ (in which tetramethylsilane was used as a standard):

$\delta$ 8.2 ppm (multiplet,1H), $\delta$ 7.6-7.2 ppm (multiplet,5H), $\delta$ 5.1 ppm (quartet, 2H)

EXAMPLE 2

Preparation of Compound (2):

2.3 g of 5-bromo-2-(2,6-dichlorobenzylthio)pyridine-N-oxide were dissolved in 50 ml of chloroform, and a solution of 1.6 g of metachloroperbenzoic acid (content: 70%) dissolved in 30 ml of chloroform was added dropwise thereto at 5 to 10°C over 10 minutes. The mixture was stirred at 5 to 10°C for an additional 2 hours. The resulting mixture was washed twice with potassium carbonate solution and dried over magnesium sulfate. The chloroform was evaporated, and the residue was recrystallized from benzene to obtain 2.0 g of 5-bromo-2-(2,6-dichlorobenzylsulfinyl)pyridine-N-oxide (Compound (2)). Yield, 83%. M.P., 187.5-188.5°C.

H-NMR in $CDCl_3$ (in which tetramethylsilane was used as a standard):

δ 8.3 ppm (multiplet, 1H), δ 7.5-7.1 ppm (multiplet, 5H), δ 5.0 ppm (quartet, 2H)

Typical examples of the compounds of this invention which can be produced in a manner similar to that described above are listed in Table 1 below.

TABLE 1

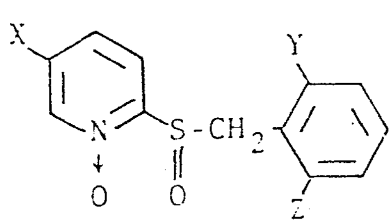

| Compound No. | X | Y | Z | Physical Constant |
|---|---|---|---|---|
| (1) | Cℓ | Cℓ | Cℓ | M.P., 176.5-177.5°C |
| (2) | Br | Cℓ | Cℓ | M.P., 187.5-188.5°C |
| (3) | Cℓ | Cℓ | F | M.P., 175.5-176.5°C |
| (4) | Br | Cℓ | F | M.P., 170-171°C |
| (5) | Cℓ | F | F | M.P., 156-157°C |
| (6) | Br | F | F | M.P., 154-155°C |

When one of the compounds of this invention is used as an active ingredient of herbicide, it can be formulated in various forms such as an emulsifiable concentrate, a wettable powder, a suspension, a dust or granules, by incorporating conventional solid carriers, liquid carriers, surfactants or other agriculturally-acceptable adjuvants. A suitable content of the compound of this invention in the composition is from 0.5 to 90% by weight, preferably from 1 to 80% by weight.

Suitable examples of the solid carriers which may be used include kaolin clay, bentonite, talc, diatomaceous earth, Jeeklite and synthetic hydrated silicon dioxide. Suitable examples of the liquid carriers which may be used include aromatic hydrocarbons (e.g., xylene or methylnaphthalene, ketones (e.g., cyclohexanone or isophorone), halogenated hydrocarbons (e.g., chlorobenzene or dichloroethane, dimethylformamide, cellosolve, ethylene glycol and water. Suitable examples of the surfactants which may be used for emulsification, dispersion or spreading include nonionic surfactants

(e.g., polyoxyethylene alkyl ethers, polyoxyethylene alkylaryl ethers, polyoxyethylene styrylaryl ethers, polyoxyethylene fatty acid esters, polyoxyethylene sorbitan fatty acid esters or oxyethyleneoxypropylene blocked polymers), and anionic surfactants (e.g., alkyl sulfates, alkyl sulfonates, dialkyl sulfosuccinates or alkylaryl sulfonates).

Suitable examples of the other agriculturally-acceptable adjuvants which may be used include lignin sulfonates, alginate, polyvinyl alcohol, cellulose, PAP (isopropyl acid phosphate), and BHT (2,6-di-t-butyl-4-methylphenol).

Typical compositions incorporating compounds of the present invention will now be given in the following Formulation Examples. In the Formulation Examples, all parts are by weight.

FORMULATION EXAMPLE 1

80 parts of Compound (2), (4) or (5), 5 parts of polyoxyethylene alkylaryl ether and 15 parts of synthetic hydrated silicon dioxide were thoroughly pulverized and mixed to obtain a wettable powder.

FORMULATION EXAMPLE 2

3 parts of Compound (1), (3) or (5), 10 parts of polyoxyethylene alkylaryl ether, 5 parts of alkylaryl sulfonate and 82 parts of isophorone were thoroughly mixed to obtain an emulsifiable concentrate.

FORMULATION EXAMPLE 3

1 part of Compound (1), (3) or (6), 1 part of synthetic hydrated silicon dioxide, 5 parts of sodium lignin sulfonate and 93 parts of kaolin clay were thoroughly

pulverized and mixed, and then well kneaded with water. The resulting mixture was granulated and dried to obtain granules.

FORMULATION EXAMPLE 4

3 parts of Compound (2), (4) or (6), 0.5 part of isopropyl acid phosphate, 66.5 parts of kaolin clay and 30 parts of talc were thoroughly pulverized and mixed to obtain dusts.

FORMULATION EXAMPLE 5

20 Parts of Compound (1), (3) or (5) were mixed with 60 parts of an aqueous solution containing 3% by weight of polyoxyethylene sorbitan monooleate, and the mixture was pulverized in a wet state until the particle size was reduced to 3 µ or less. The resulting mixture was mixed with 20 parts of an aqueous solution containing 3% by weight of sodium alginate as a dispersion stabilizer to obtain a suspension.

The thus-obtained emulsifiable concentrate, wettable powders, suspensions and the like were, in general, used for foliar treatment or soil treatment after dilution with water. On the other hand, the granules, dusts and the like were used for the same treatments as they were. In order to strengthen their activity as herbicides, the compounds of this invention may be applied together with other herbicides with or without being mixed therewith, and in some cases, a synergistic effect may be expected. Further, the compounds of this invention may be mixed or used together with one or more of insecticides, acaricides, nematocides, fungicides, plant growth regulators, fertilizers and soil improvers.

The compounds of this invention may also be used as the active ingredient of herbicides in orchards, pasture lands, turf lands, forests and various non-agricultural lands in addition to upland fields and paddy fields.

For any instance of foliar treatment or soil treatment, the amount of the compound of this invention which is applied can be altered within a considerably wide range.

However, it is usually from 0.1 to 200 g, preferably from 0.2 to 100 g, per are calculated as the amount of active ingredient. The concentration is from 0.02 to 2% by weight for application as a water-diluted solution of emulsifiable concentrate, wettable powder or suspension. The amount applied and concentration of the compound may be altered depending upon the form of composition, the place of application, the method of application, the time of application, the kind of crop, the kind of weed, the degree of damage by weeds and the climatic conditions, regardless of the range given above.

In order to demonstrate that the compounds of this invention are useful as active ingredients of herbicides, the invention will now be illustrated by reference to the following Test Examples. In the Test Examples, the phytotoxicity against crops and the herbicidal activity against weeds were evaluated on a scale of 0 to 5 grades by weighing the fresh weight of the aerial parts of the remaining withered plants and calculating the ratio (%) to that of the untreated plot. The evaluation was made in accordance with the criteria shown in Table 2 below.

TABLE 2

| Evaluation Grade | Ratio (%) of Fresh Weight to That of Untreated Plot | |
|---|---|---|
| | Herbicidal Activity against Weeds | Phytotoxicity against Crops |
| 0 | 91~ | 91~ |
| 1 | 71~90 | 71~90 |
| 2 | 41~70 | 51~70 |
| 3 | 11~40 | 31~50 |
| 4 | 4~10 | 11~30 |
| 5 | 0 ~ 3 | 0~10 |

Comparative Compounds used in the Test Examples are listed in Table 3 below.

TABLE 3

| Compound No. | Chemical Structure | Physical Constant (melting point) (°C) |
|---|---|---|
| (a) | 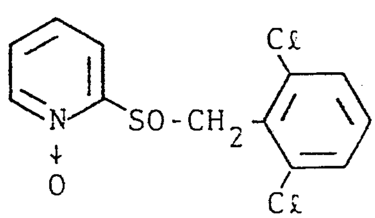 | 135-137 |
| (b) | 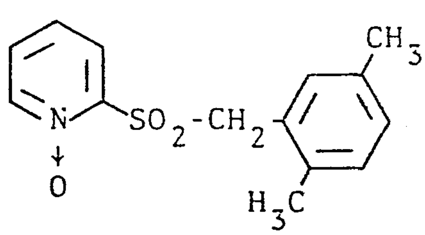 | 152-154 |

(cont'd)

| Compound No. | Chemical Structure | Physical Constant (melting point) (°C) |
| --- | --- | --- |
| (c) | 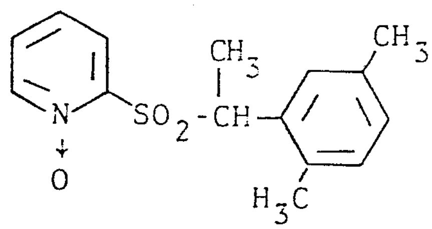 | 162-163 |
| (d) | 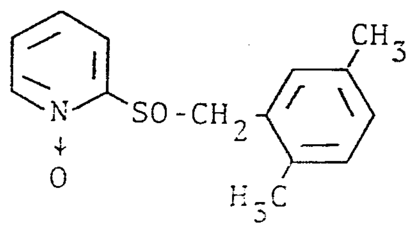 | 124-125 |
| (e) | 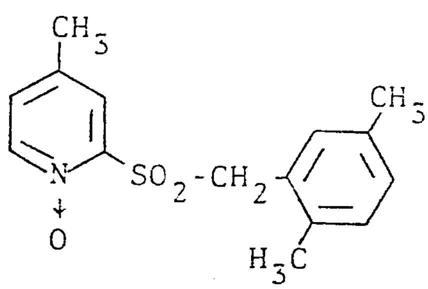 | 134-135 |
| (f) | 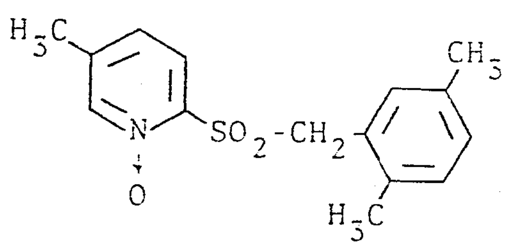 | 154.5-156 |
| (g) | 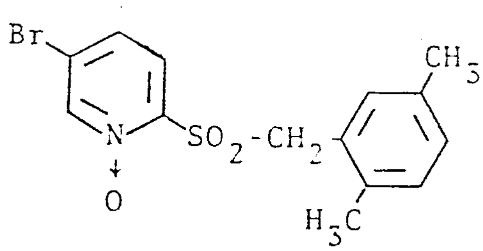 | 185-186 |

(cont'd)

| Compound No. | Chemical Structure | Physical Constant (melting point) (°C) |
|---|---|---|
| (h) | 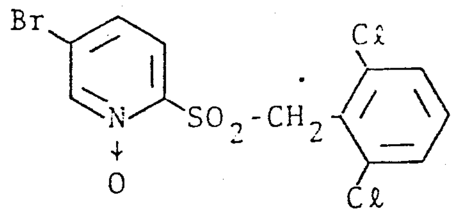 | 207-209 |
| (i) | 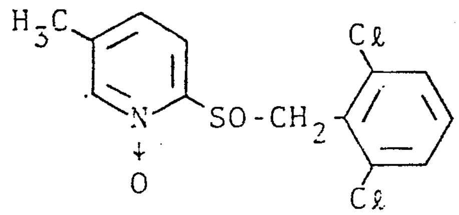 | 153-154 |

Compounds (a) to (c): Compounds as disclosed in U.S. Patent 4,019,893

Compounds (d) to (h): Compounds as disclosed in European Patent Publication No. 0036638A2

Compound (i): Compound as produced for comparison

## TEST EXAMPLE 1

Pre-Emergence Soil Treatment in Paddy Field

A plastics pot having an inside diameter of 8 cm and a height of 10 cm was packed with paddy field soil and predetermined amounts of seeds of barnyyard grass (*Echinochloa oryzicola*), broad-leafed weeds (false pimpernel (*Lindernia procumbens*) and spike-flowered rotala (*Rotala indica*) and bulrush (*Scirpus juncoides*) were sown, followed by covering with soil. Then, water was poured into the pot to a water depth of 3 cm. The resulting prepared pot was allowed to stand in a greenhouse for one day, and a solution prepared by diluting a predetermined amount of an emulsifiable concentrate formulated in accordance with Formulation Example 2 with 5 ml of water was added dropwise onto the water surface.

The pot was allowed to stand in the greenhouse for an additional 20 days to evaluate the herbicidal activity. The results obtained are shown in Table 4 below.

TABLE 4

| Compound No. | Application Amount of Active Ingredient (g/a) | Herbicidal Activity | | |
|---|---|---|---|---|
| | | Barnyard-grass | Broad-Leafed Weeds | Bulrush |
| (1) | 20 | 5 | 5 | 5 |
| | 5 | 5 | 5 | 5 |
| (2) | 20 | 5 | 5 | 5 |
| | 5 | 5 | 5 | 5 |
| (3) | 20 | 5 | 5 | 5 |
| | 5 | 5 | 5 | 4 |
| (4) | 20 | 5 | 5 | 5 |
| | 5 | 5 | 5 | 4 |
| (5) | 20 | 5 | 5 | 5 |
| | 5 | 5 | 5 | 5 |
| (6) | 20 | 5 | 5 | 5 |
| | 5 | 5 | 5 | 5 |

(cont'd)

| Compound No. | Application Amount of Active Ingredient (g/a) | Herbicidal Activity | | |
|---|---|---|---|---|
| | | Barnyard-grass | Broad-Leafed Weeds | Bulrush |
| (a) | 20 | 4 | 4 | 3 |
| | 5 | 3 | 3 | 2 |
| (b) | 20 | 3 | 2 | 1 |
| | 5 | 2 | 0 | 1 |
| (c) | 20 | 2 | 0 | 0 |
| | 5 | 0 | 0 | 0 |
| (d) | 20 | 5 | 3 | 2 |
| | 5 | 3 | 2 | 1 |
| (e) | 20 | 5 | 4 | 2 |
| | 5 | 3 | 3 | 2 |
| (f) | 20 | 2 | 0 | 2 |
| | 5 | 1 | 0 | 2 |
| (g) | 20 | 3 | 4 | 5 |
| | 5 | 2 | 2 | 3 |
| (h) | 20 | 3 | 4 | 3 |
| | 5 | 1 | 4 | 2 |
| (i) | 20 | 5 | 3 | 3 |
| | 5 | 4 | 2 | 2 |

## TEST EXAMPLE 2

Foliar and Soil Treatment in Paddy Field

A 1/5,000 are Wagner pot was packed with paddy field soil, and predetermined amounts of seeds of barnyyard grass (*Echinochloa oryzicola*), bulrush (*Scirpus juncoides*) and broad-leafed weeds (false pimpernel (*Lindernia procumbens*) and spike-flowered rotala (*Rotala indica*)) and hibernated buds of slender spikerush (*Eleocharis acicularis*) were incorporated into the soil at a depth of 3 cm from the soil surface. Water was then added to the pot to a water depth of 4 cm; 3-leaf stage rice (*Oryza sativa*) was then transplanted. The plants were grown in a greenhouse for 5 days, and when the weeds had germinated, a solution prepared by diluting a predetermined amount of an emulsifiable concentrate formulated in accordance with Formulation Example 2 with 10 ml of water was added dropwise onto the water surface. 20 days after the addition, the herbicidal activity and the phytotoxicity against the rice were evaluated. The results obtained are shown in Table 5 below.

TABLE 5

| Compound No. | Application Amount of Active Ingredient (g/a) | Phyto-toxicity against Crop Rice | Herbicidal Activity A | B | C | D |
|---|---|---|---|---|---|---|
| (1) | 10 | 1 | 5 | 5 | 5 | 5 |
| | 5 | 0 | 5 | 5 | 5 | 5 |
| (2) | 10 | 1 | 5 | 5 | 5 | 5 |
| | 5 | 1 | 4 | 5 | 5 | 5 |
| (3) | 10 | 1 | 5 | 5 | 5 | 5 |
| | 5 | 0 | 5 | 5 | 5 | 5 |
| (5) | 10 | 1 | 5 | 5 | 5 | 5 |
| | 5 | 1 | 5 | 5 | 5 | 5 |
| (6) | 10 | 1 | 5 | 5 | 5 | 5 |
| | 5 | 0 | 4 | 5 | 5 | 5 |
| (a) | 10 | 2 | 4 | 4 | 4 | 3 |
| | 5 | 1 | 3 | 3 | 2 | 2 |
| (c) | 10 | 2 | 3 | 2 | 2 | 2 |
| | 5 | 1 | 3 | 0 | 1 | 2 |
| (h) | 10 | 2 | 2 | 4 | 3 | 3 |
| | 5 | 1 | 1 | 4 | 2 | 2 |
| (i) | 10 | 3 | 4 | 3 | 4 | 3 |
| | 5 | 2 | 3 | 2 | 2 | 2 |

A: Barnyardgrass

B: Broad-Leafed Weeds

C: Slender Spikerush

D: Bulrush

## TEST EXAMPLE 3

Pre-Emergence Soil Incorporation Treatment in Upland Field

A cylindrical plastics pot having a diameter of 10 cm and a height of 10 cm was packed with upland field soil and predetermined amounts of seeds of Japanese millet (Echinochloa frumentacea), oat (Avena sativa), tall morning glory (Ipomoea purpurea) and velvetleaf (Abutilon theophrasti) and tubers of yellow nutsedge (Cyperus esculentus) were sown, followed by covering with soil. Then, a solution prepared by diluting an emulsifiable concentrate formulated in accordance with Formulation Example 2 with water was applied onto the surface of the soil in a spray volume of 10 l per are by means of a small-sized sprayer. Thereafter, the surface portion of soil up to a depth of 4 cm was thoroughly incorporated. The plants were then grown in a greenhouse for 20 days, and the herbicidal activity was evaluated. The results obtained are shown in Table 6 below.

TABLE 6

| Compound No. | Application Amount of Active Ingredient (g/a) | Herbicidal Activity A | B | C | D | E |
|---|---|---|---|---|---|---|
| (1) | 80 | 5 | 5 | 5 | 5 | 5 |
| | 20 | 5 | 4 | 5 | 4 | 5 |
| (2) | 80 | 5 | 5 | 4 | 5 | 5 |
| | 20 | 4 | 4 | 3 | 3 | 5 |
| (3) | 40 | 5 | 5 | 5 | 5 | 5 |
| | 10 | 5 | 5 | 4 | 4 | 5 |
| (4) | 80 | 5 | 5 | 4 | 5 | 5 |
| | 20 | 4 | 4 | 3 | 3 | 4 |
| (5) | 40 | 5 | 5 | 5 | 5 | 5 |
| | 10 | 5 | 5 | 4 | 4 | 5 |
| (6) | 80 | 5 | 5 | 5 | 5 | 5 |
| | 20 | 5 | 5 | 3 | 3 | 5 |

A: Japanese millet

B: Oat

C: Tall Morning glory

D: Velvetleaf

E: Yellow nutsedge

TEST EXAMPLE 4

Soil Treatment in Upland Field

A flat (area: 33 x 23 $cm^2$, heigh: 11 cm) was packed with an upland field soil, and predetermined amounts of seeds of soybeans (Glycine max), cotton (Gosspium sp.), bermudagrass (Cynodon dactylon), goosegrass (Eleusine indica), barnyardgrass (Echinochloa crus-galli), green foxtail (Setaria viridis) and fall panicum (Panicum dichotomiflorum) were sown, followed by covering with the soil to a thickness of 1 to 2 cm. A solution prepared by diluting a predetermined amount of an emulsifiable concentrate formulated in accordance with Formulation Example 2 with water was applied onto the surface of the soil in a proportion of 5 l per are by means of a small-sized sprayer. After the application, the plants were grown in a greenhouse for 20 days, and the herbicidal activity was evaluated. The results obtained are shown in Table 7 below.

TABLE 7

| Compound No. | Application Amount of Active Ingredient (g/a) | Herbicidal Activity A | B | C | D | E | F | G |
|---|---|---|---|---|---|---|---|---|
| (1) | 1 | 0 | 0 | 5 | 5 | 5 | 5 | 5 |
| | 0.5 | 0 | 0 | 4 | 4 | 3 | 4 | 4 |
| (3) | 1 | 0 | 0 | 5 | 5 | 5 | 5 | 5 |
| | 0.5 | 0 | 0 | 5 | 4 | 4 | 4 | 5 |
| (4) | 1 | 0 | 0 | 5 | 5 | 4 | 5 | 5 |
| | 0.5 | 0 | 0 | 4 | 3 | 4 | 4 | 5 |
| (5) | 1 | 0 | 0 | 5 | 5 | 5 | 5 | 5 |
| | 0.5 | 0 | 0 | 4 | 5 | 4 | 4 | 5 |
| (6) | 1 | 0 | 0 | 5 | 5 | 4 | 4 | 5 |
| | 0.5 | 0 | 0 | 4 | 3 | 4 | 3 | 4 |
| (a) | 1 | 0 | 0 | 3 | 4 | 3 | 4 | 4 |
| | 0.5 | 0 | 0 | 1 | 2 | 1 | 2 | 2 |
| (h) | 1 | 0 | 0 | 3 | 2 | 0 | 1 | 2 |
| | 0.5 | 0 | 0 | 0 | 0 | 0 | 0 | 1 |

A: Soybeans

B: Cotton

C: Bermudagrass

D: Goosegrass

E: Barnyardgrass

F: Green Foxtail

G: Fall Panicum

TEST EXAMPLE 5

Soil Treatment in Upland Field

A flat (area: 33 x 23 $cm^2$, height: 11 cm) was packed with upland field soil, and predetermined amounts of seeds of barley (Hordeum vulgare), quackgrass (Agropyron repens), wild oat (Avena fatua), black grass (Alopecurus myosuroides) and annual bluegrass (Poa annua) were sown, followed by covering with soil to a thickness of 1 to 2 cm. A solution prepared by diluting a predetermined amount of an emulsifiable concentrate formulated in accordance with Formulation Example 2 with water was applied onto the surface of the soil in a spray volume of 5 l per are by means of a sprayer. After the application, the plants were grown in a greenhouse for 27 days, and the herbicidal activity was evaluated. The results obtained are shown in Table 8 below.

TABLE 8

| Compound No. | Application Amount of Active Ingredient (g/a) | Herbicidal Activity A | B | C | D | E |
|---|---|---|---|---|---|---|
| (3) | 3 | 1 | 5 | 5 | 5 | 5 |
| | 1.5 | 0 | 4 | 4 | 5 | 5 |
| (5) | 3 | 0 | 4 | 5 | 5 | 5 |
| | 1.5 | 0 | 4 | 4 | 5 | 5 |
| (a) | 3 | 1 | 1 | 2 | 4 | 4 |
| | 1.5 | 0 | 0 | 0 | 3 | 3 |
| (h) | 3 | 0 | 0 | 1 | 2 | 3 |
| | 1.5 | 0 | 0 | 0 | 1 | 1 |

A: Barley

B: Quackgrass

C: Wild Oat

D: Black Grass

E: Annual Bluegrass

Claims:

1. A benzylsulfinyl pyridine-N-oxide of the following general formula (I):

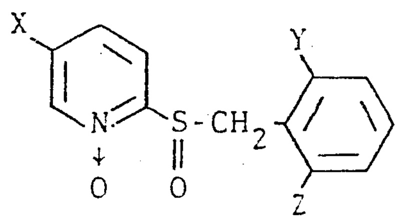

(I)

wherein X is a chlorine atom or a bromine atom, and Y and Z, which may be the same or different, are each a chlorine atom or a fluorine atom.

2. 5-Chloro-2-(2-chloro-6-fluorobenzyl-sulfinyl)pyridine-N-oxide.

3. A process for preparing a benzylsulfinyl pyridine-N-oxide of the general formula (I) as set forth and defined in Claim 1, characterised in that a benzylthiopyridine-N-oxide of the following general formula (II):

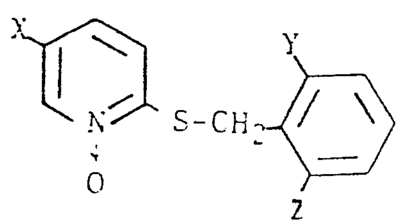

(II)

wherein X, Y and Z are each as defined in Claim 1, is oxidized with an oxidizing agent.

4. A herbicidal composition which comprises, as an active ingredient, a herbicidally effective amount of a benzylsulfinyl pyridine-N-oxide as claimed in Claim 1, and an inert carrier.

5. A method for controlling weeds which comprises applying a herbicidally effective amount of a benzylsulfinyl pyridine-N-oxide as claimed in Claim 1 to an existing or potential weed-infested area.

6. A method according to Claim 5, wherein the area is for the cultivation of soybeans, cotton, barley, or rice.

7. Use of a benzylsulfinyl pyridine-N-oxide as claimed in Claim 1 as a herbicide.